# EUROPEAN PATENT SPECIFICATION

(11) **EP 3 342 851 B1**
(45) Date of publication and mention of the grant of the patent: **30.11.2022**
(21) Application number: 16839366.8
(22) Date of filing: 26.08.2016
(51) Int. Cl.: C12Q 1/68, C12Q 1/6834, C12M 1/34, G01N 33/53, G01N 33/543, C12N 15/09

(54) **NUCLEIC ACID DETECTION DEVICE AND NUCLEIC ACID DETECTION METHOD**
NUKLEINSÄUREDETEKTIONSVORRICHTUNG UND NUKLEINSÄUREDETEKTIONSVERFAHREN
DISPOSITIF DE DÉTECTION D'ACIDE NUCLÉIQUE ET PROCÉDÉ DE DÉTECTION D'ACIDE NUCLÉIQUE

(30) Priority: 26.08.2015 JP 2015167198
(43) Date of publication of application: 04.07.2018
(73) Proprietor: Kaneka Corporation, Osaka-shi, Osaka 530-8288 (JP)
(72) Inventor: NISHIZONO Takashi, Takasago-shi Hyogo 676-8688 (JP); SANO Sotaro, Takasago-shi Hyogo 676-8688 (JP); MIYAMOTO Shigehiko, Takasago-shi Hyogo 676-8688 (JP); TANAKA Hozumi, Takasago-shi Hyogo 676-8688 (JP)
(74) Representative: Vossius & Partner Patentanwälte Rechtsanwälte mbB
(86) International application number: PCT/JP2016/074953
(87) International publication number: WO 2017/034019

(56) References cited:
- WO-A1-2009/027932
- WO-A1-2011/112068
- WO-A1-2013/122453
- WO-A1-2014/108322
- WO-A2-2007/116298
- JP-A- 2006 201 062
- JP-A- 2012 528 583
- JP-A- 2013 106 549
- US-A1- 2005 227 275
- US-A1- 2011 229 887

## Description

### Technical Field

The present invention relates to a nucleic acid detection device and a nucleic acid detection method.

### [Background Art]

With progression of genetic engineering, genetic tests have been used in detection of pathogens or cancer cells, the analysis of the cell kinetics, constitution inspections, tests regarding the effectiveness of pharmaceutical products, etc. The genetic test is generally composed of a step of collecting a specimen, a step of preparing a nucleic acid from the specimen, a nucleic acid amplification reaction step and/or a nucleic acid labeling reaction step, and a step of detecting the amplified and/or labeled nucleic acid.

Regarding the detection step, nucleic acid detection devices, such as lateral flow-type (Patent Literature 1), dip stick-type (Patent Literature 2) and array-type (Patent Literature 3) nucleic acid detection devices, which are capable of capturing and/or detecting the amplified and/or labeled nucleic acid on a solid phase carrier, have been developed and utilized. In general, these nucleic acid detection devices each comprise a target detection portion for detecting the amplified and/or labeled reaction product of the target nucleic acid, and a control detection portion for detecting the amplified and/or labeled reaction product of a control nucleic acid (internal standard substance) that has been added as a control in advance. The control detection portion is used to evaluate whether the amplification reaction and/or labeling reaction have normally progressed. Specifically, detecting signals in the control detection portion can evaluate whether reagents such as enzymes have functioned normally and whether experimental operations have been implemented appropriately without problems, and the reliability of the test results can be confirmed.

US 2011/229887 A1 discloses methods and compositions for confirming success of an amplification reaction involving a target nucleic acid and a control nucleic acid. Test nucleic acids are immobilized at multiple locations, such that amplification of either a test nucleic acid or a control nucleic acid provides a captured nucleic acid in a control capture zone. In particular, biotin-labelled test nucleic acids are captured in a control and a test capture zone using immobilized antibodies.

US 2005/227275 A1 discloses a nucleic acid detection system and more specifically a signal amplification system using, e.g., europium encapsulated microparticles and/or electroconductivity in conjunction with a chromatographic lateral flow assay.

### Citation List

### Patent Literature

Patent Literature 1: JP Patent Publication (Kokai) No. 2013-247968 A
Patent Literature 2: JP Patent Publication (Kokai) No. 2014-057565 A
Patent Literature 3: JP Patent Publication (Kokai) No. 2004-502929 A

### Summary of Invention

### Technical Problem

Regarding conventional nucleic acid detection devices, the present inventors have thought that signals, which should have been obtained in the control detection portion of such a conventional nucleic acid detection device, are likely to disappear or decrease, even when there are no problems regarding reagents or experimental operations.

### Solution to Problem

The present inventors have surprisingly elucidated that when large quantities of target nucleic acids are present in a reaction system, for example, in multiplex PCR, etc., there is a case where the target nucleic acids are preferentially amplified, and the amplification reaction and/or labeling reaction of control nucleic acids are inhibited, so that signals from a control detection portion would disappear or weaken.

Moreover, the present inventors have found that when not only a control nucleic acid, but also at least one type of target nucleic acid is captured and/or detected in the control detection portion of a nucleic acid detection device, the disappearance or reduction of signals from the control detection portion can be prevented, and high signals can be stably obtained. Furthermore, the present inventors have provided a nucleic acid detection device utilizing this finding, thereby completed the present invention.

Specifically, the present invention is as follows.

(1) A nucleic acid detection device comprising at least one target detection portion and a control detection portion, wherein a probe for capturing a target nucleic acid is immobilized on the target detection portion, and a probe for capturing a control nucleic acid and at least one probe for capturing the target nucleic acid are immobilized on the control detection portion, wherein the target nucleic acid and the control nucleic acid are amplification reaction products that have undergone an amplification step and wherein the probe is a nucleic acid.
(2) In a more preferred embodiment of the nucleic acid detection device according to the above (1), the device is a chromatographic device.
(3) A nucleic acid detection kit comprising the nucleic acid detection device according to the above (1) or (2), a control template nucleic acid, a nucleic acid amplification enzyme, a nucleic acid amplification reaction reagent, and a nucleic acid detection reaction reagent.
(4) A nucleic acid detection method for detecting at least one target nucleic acid and a control nucleic acid, which comprises the following steps (a) to (d):
   (a) a step of simultaneously performing a nucleic acid amplification reaction in a single reaction vessel, using primers, a template nucleic acid and a nucleic acid amplification reaction reagent, so as to amplify the at least one target nucleic acid and the control nucleic acid,
   (b) a step of allowing the at least one target nucleic acid to bind to a probe immobilized on a target detection portion on a solid phase carrier,
   (c) a step of allowing the at least one target nucleic acid and the control nucleic acid to bind to at least one probe immobilized on a control detection portion on the solid phase carrier, and
   (d) a step of labeling the captured target nucleic acid and control nucleic acid, and detecting signals derived from the target nucleic acid and the control nucleic acid, wherein the probe is a nucleic acid.

The present description includes the contents as disclosed in Japanese Patent Application No. 2015-167198, which is a priority document of the present application.

### Advantageous Effects of Invention

According to the present invention, since the disappearance or reduction of signals from a control detection portion in a nucleic acid detection device can be prevented and high signals can be stably obtained, the reliability of test results can be easily confirmed.

### Brief Description of Drawings

[Figure 1] Figure 1 is a figure (plan view) showing a lateral flow-type device of the present invention.
[Figure 2] Figure 2 is a figure showing a dip stick-type device of the present invention.
[Figure 3] Figure 3 is a figure showing an array-type device of the present invention.
[Figure 4] Figure 4 is a figure showing Example 2 of the present invention.
[Figure 5] Figure 5 is a figure showing Comparative Examples 2 and 8 of the present invention.
[Figure 6] Figure 6 is a figure showing Example 4 of the present invention.
[Figure 7] Figure 7 is a figure showing Comparative Example 4 of the present invention.
[Figure 8] Figure 8 is a figure showing Example 5 of the present invention.
[Figure 9] Figure 9 is a figure showing Comparative Example 5 of the present invention.
[Figure 10] Figure 10 is a figure showing Example 7 of the present invention.
[Figure 11] Figure 11 is a figure showing Comparative Example 7 of the present invention.
[Figure 12] Figure 12 is a figure showing Example 8 of the present invention.
[Figure 13] Figure 13 is a figure (cross-sectional view) showing a lateral flow-type device of the present invention.

### Description of Embodiments

Preferred aspects of collection of a specimen, preparation of a nucleic acid from the specimen, amplification of the nucleic acid, and detection of the nucleic acid, which can be used in the nucleic acid detection method of the present invention, will be described below.

### < Step of collecting specimen >

The specimen used as an analyte, in which the presence of a target nucleic acid is to be detected, is not particularly limited, as long as it is a sample possibly containing the nucleic acid. Examples of such a specimen include animal or plant cells, tissues, whole blood, serum, body fluids such as lymph fluid, bone marrow fluid, tissue fluid, urine, sperm, vaginal fluid, amniotic fluid, tear, saliva, sweat or milk juice, cell-derived vesicles such as exosome, feces, throat swab, phlegm, bacteria, virus, and viroid. For collection of a specimen, various types of conventionally known methods can be used.

The nucleic acid used in the present invention is broadly classified into natural nucleic acid and non-natural nucleic acid. The "natural nucleic acid" is a polynucleotide comprising nucleotides as basic portions, wherein the nucleotides are bound to one another by a phosphodiester bond that is a linkage between the 3' carbon atom of one sugar molecule and the 5' carbon atom of another sugar. Examples of the natural nucleic acid include deoxyribonucleotides such as DNA or RNA, and polymers of ribonucleotides. The "non-natural nucleic acid" is a nucleic acid comprising non-natural nucleotides, instead of or in addition to the above-described natural nucleotides. The non-natural nucleotide indicates a nucleotide, the basic portion or other portion of which has been artificially modified, or an artificially produced nucleotide analog having properties similar to those of a nucleotide. Examples of the non-natural nucleotide include xanthosines and diaminopyrimidines.

The target nucleic acid used in the present invention is a nucleic acid to be detected. The present target nucleic acid is not particularly limited, as long as it comprises a target sequence. Examples of the present target nucleic acid include the aforementioned specimen-derived genomic DNA, plasmid DNA, ctDNA (cfDNA), mRNA, miRNA, IncRNA, and an amplicon. In the present description, an amplification reaction product obtained by amplification of a target nucleic acid contained in a specimen may also be referred to as a "target nucleic acid."

The control nucleic acid used in the present invention is a nucleic acid used as an internal standard for confirming that operations or reagents are not problematic upon detection of a target nucleic acid. The present control nucleic acid is not particularly limited, as long as it exhibits the aforementioned function. In the present invention, an amplification reaction product obtained by amplification of a control nucleic acid added as a template nucleic acid to a nucleic acid amplification reaction system may also be referred to as a "control nucleic acid."

### < Step of preparing nucleic acid from specimen >

In a step of preparing a nucleic acid used as a template nucleic acid in the below-described step (a) from a specimen, a nucleic acid may be extracted, or separated and/or purified from a specimen. The means are not particularly limited. Examples of the extraction method include physical disintegration such as the use of heat or ultrasonic waver, disintegration using drugs such as an alkaline reagent, an organic solvent or a surfactant, and disintegration using enzymes such as lysozyme or proteinase K. Examples of the separation and/or purification include a bind-elute method, a treatment using an ion exchange resin such as zeolite, and centrifugation. However, the step of preparing a nucleic acid from a specimen is not particularly limited, as long as the prepared nucleic acid can be used as a template nucleic acid and the subsequent biochemical reactions such as a nucleic acid amplification reaction can progress without problems. Moreover, it is also possible to omit the present step and to directly subject a specimen as a supply source of a template nucleic acid to the subsequent nucleic acid amplification reaction.

### < Step of amplifying nucleic acid (step (a)) >

Step (a) of the method of the present invention is a step of using primers, a template nucleic acid and a nucleic acid amplification reaction reagent (nucleic acid amplification reagent) to simultaneously perform a nucleic acid amplification reaction in a single reaction vessel, so as to amplify the above-described at least one target nucleic acid and the above-described control nucleic acid.

In step (a), the template nucleic acid comprises nucleic acids possibly including target nucleic acids (typically, nucleic acids derived from the above-described specimen) and a control nucleic acid. The control nucleic acid, which is to be added as a template nucleic acid to the nucleic acid amplification reaction system, is also referred to as a "control template nucleic acid."

The phrase "simultaneously perform a nucleic acid amplification reaction in a single reaction vessel" is used to mean that the nucleic acid amplification reaction of the above-described at least one target nucleic acid and the nucleic acid amplification reaction of the above-described control nucleic acid are simultaneously performed in a single reaction vessel. Hence, primers used in step (a) include a primer set capable of amplifying each of one or more target nucleic acids and a primer set capable of amplifying a control nucleic acid.

Step (a) is preferably a step of performing a nucleic acid amplification reaction, using a set of tagged primers as the above-described primers, so as to prepare an amplification reaction product, to which two types of tags have been added.

The tagged primer means a primer used in nucleic acid amplification, to which a tag is added. This tagged primer can be used for the purpose of preparing a tagged amplification reaction product.

The primer used in the present invention is a single-stranded nucleic acid consisting of 5 to 80 nucleotides, which specifically recognizes a target nucleic acid or a control nucleic acid and serves as an origin of elongation in a nucleic acid amplification reaction. Specifically, the nucleotide sequence of each primer is a sequence hybridizable with the 3'-terminal side of the target nucleotide sequence (the nucleotide sequence as an amplification target) of a target nucleic acid or a control nucleic acid, or with the 3'-terminal side of the complementary nucleotide sequence of the target nucleotide sequence, and in general, it is a nucleotide sequence complementary to the nucleotide sequence of the 3-terminal side of the target nucleotide sequence, or a nucleotide sequence complementary to the 3'-terminal side of the complementary nucleotide sequence of the target nucleotide sequence. Herein, the "target nucleotide sequence" means a nucleotide sequence to be detected, or a complementary nucleotide sequence thereof. When the target nucleic acid or control nucleic acid is a double-stranded nucleic acid, the target nucleotide sequence means the nucleotide sequence of either one strand of the double-stranded nucleic acid. As long as the primer can specifically bind to a target nucleic acid or a control nucleic acid, the primer may have a deletion or insertion of nucleotides, and a mismatch portion. Herein, the "3'-terminal side" of a predetermined nucleotide sequence means a partial nucleotide sequence consisting of a plurality of consecutive nucleotides (typically, 5 to 80 nucleotides) comprising the nucleotide at the 3'-terminus of the predetermined nucleotide sequence.

In the present invention, the phrase "nucleotide sequence X is 'hybridizable' with nucleotide sequence Y" means that a polynucleotide (in particular, DNA) comprising the nucleotide sequence X is hybridized with a polynucleotide (in particular, DNA) comprising the nucleotide sequence Y under stringent conditions, and that the polynucleotide comprising the nucleotide sequence X is not hybridized with a polynucleotide that does not comprise the nucleotide sequence Y. That is to say, the term "hybridize" means specific hybridization. Herein the "stringent conditions" mean conditions in which, what is called, a specific hybrid is formed and a non-specific hybrid is not formed. The stringent conditions can be determined depending on, for example, the melting temperature Tm (°C) of the primer used in the present invention and a complementary strand thereof, and the salt concentration in a hybridization solution. Such stringent conditions can be determined with reference to, for example, Green and Sambrook, Molecular Cloning, 4th Ed (2012), Cold Spring Harbor Laboratory Press. Specifically, the stringent conditions can be determined based on the temperature and the salt concentration in a solution, which are applied during Southern hybridization, and the temperature and the salt concentration in a solution, which are applied during the washing step of Southern hybridization. More specifically, the stringent conditions, which are applied, for example, in a hybridization step, are a sodium concentration of 25 to 500 mM, and preferably 25 to 300 mM, and a temperature that is slightly lower than Tm determined from the polynucleotide sequence (e.g., a temperature that is 0°C to approximately 5°C lower than Tm), for example, 40°C to 68°C, and preferably 40°C to 65°C. Further specifically, hybridization can be carried out at 1 to 7 × SSC, at 0.02% to 3% SDS, and at a temperature of 40°C to 60°C. Moreover, after completion of the hybridization, a washing step may be carried out. Such a washing step can be carried out, for example, at 0.1 to 2 × SSC, at 0.1% to 0.3% SDS, and at a temperature of 50°C to 65°C.

Primers for amplifying a target nucleic acid are configured to generate a target nucleic acid, to which a labeling tag and a tag for binding to a solid phase carrier bind, as an amplification reaction product. Accordingly, a pair of tagged primers can be used herein as primers. In addition, in a case where a tag is not added to either one of or both of a pair of primers, a nucleic acid amplification reaction reagent comprising a tagged nucleotide source can be used, such that the amplification reaction of a target nucleic acid can generate an amplification reaction product comprising tagged nucleotides.

Similarly, primers for amplifying a control nucleic acid are configured to generate a control nucleic acid, to which a labeling tag and a tag for binding to a solid phase carrier bind, as an amplification reaction product. Accordingly, a pair of tagged primers can be used herein as primers. In addition, in a case where a tag is not added to either one of or both of a pair of primers, a nucleic acid amplification reaction reagent comprising a tagged nucleotide source can be used, such that the amplification reaction of a control nucleic acid can generate an amplification reaction product comprising tagged nucleotides.

Herein, the "labeling tag" means a tag capable of binding to the below-described labeled probe. On the other hand, the "tag for binding to a solid phase carrier" means a tag capable of binding to a probe immobilized on the below-described solid phase carrier.

The tag is a substance used to label an amplification reaction product or a substance used to bind an amplification reaction product to a solid phase, wherein the substance does not generate signals by itself. Examples of such a tag include a nucleic acid, a biotin, a hapten such as DIG (digoxigenin) or FITC (fluorescein isothiocyanate), and a sugar chain. When a single-stranded nucleic acid is used as a tag, it is preferable to insert a spacer structure consisting of a polymerase reaction inhibition region between a tag and a primer, such that the nucleic acid cannot be converted to a double-stranded nucleic acid by a nucleic acid amplification reaction. The spacer structure consisting of a polymerase reaction inhibition region is not particularly limited, as long as it is able to inhibit a nucleic acid elongation reaction by polymerase and is able to keep the single-stranded structure of the tag. Examples of the spacer structure include insertion of various types of natural or non-natural modifications, such as azobenzene modification, alkylene chain or polyoxyalkylene chain modification, and inverted nucleotide modification. The number of nucleotides in the nucleic acid used as a tag is not particularly limited. The number of nucleotides can be, for example, 5 to 80. Examples of the tagged nucleotide source include dNTPs (dUTP, dCTP, etc.) to which biotin or the above-described hapten is bound.

The nucleic acid amplification reaction used in the present invention includes a PCR method as a typical example. The present nucleic acid amplification reaction is not particularly limited, as long as it amplifies a specific nucleic acid sequence. In addition to the PCR method, examples of the nucleic acid amplification reaction include known methods such as LCR (Ligase Chain Reaction) method, SDA (Strand Displacement Amplification) method, RCA (Rolling Circle Amplification) method, CPT (Cycling Probe Technology) method, Q-Beta Replicase Amplification Technology method, ICAN (Isothermal and Chimeric primer-initiated Amplification of Nucleic Acids) method, LAMP (Loop-Mediated Isothermal Amplification of DNA) method, NASBA (Nucleic acid Sequence-based Amplification method) method, TMA (Transcription mediated amplification method) method, RPA (Recombinase Polymerase Amplification) method, and SIBA (Strand Invasion Based Amplification) method. The Q-Beta Replicase Amplification Technology method, the RCA method, the NASBA method, the SDA method, the TMA method, the LAMP method, the ICAN method, the RPA method, the SIBA method and the like are methods in which an amplification reaction is carried out at a constant temperature. Other methods, such as the PCR method or the LCR method, are methods in which an amplification reaction is carried out in a temperature cycling system.

The nucleic acid amplification reaction reagent used in the nucleic acid amplification reaction comprises a nucleic acid amplification enzyme and other components necessary for the nucleic acid amplification reaction.

The enzyme used in the nucleic acid amplification reaction is not particularly limited, and commercially available polymerase or other enzymes can be preferably used. Examples of the nucleic acid amplification enzyme include *E*. *coli*-derived DNA polymerase I, T4 DNA polymerase, T7 DNA polymerase, Taq DNA polymerase, KOD DNA polymerase, Pfu DNA polymerase, Bst DNA polymerase, Bsu DNA polymerase, Phi29 DNA polymerase, Bca BEST DNA polymerase, reverse transcriptase, SP6 RNA polymerase, T7 RNA polymerase, and T3 RNA polymerase, but are not limited thereto.

Other components comprised in the nucleic acid amplification reaction reagent will be described later.

In the above-described step (a), at least one target nucleic acid (in a case where target nucleic acids are present in the template nucleic acids) and a control nucleic acid, to each of which two types of tags have been added, are obtained as nucleic acid amplification products. In each target nucleic acid or control nucleic acid, the above-described two types of tags are a labeling tag and a tag used for binding to a solid phase carrier, as described above. The nucleic acid amplification products of each target nucleic acid and control nucleic acid obtained in step (a) are generally nucleic acid amplification products, in which each target nucleic acid or control nucleic acid has been double-stranded, and the above-described two types of tags have been added thereto.

### < Steps of detecting nucleic acid (Steps (b), (c) and (d)) >

The nucleic acid detection method of the present invention comprises the following step (b), (c) and (d), in addition to the above described step (a):
(b) a step of allowing the at least one target nucleic acid to bind to a probe immobilized on a target detection portion on a solid phase carrier,
(c) a step of allowing the at least one target nucleic acid and the control nucleic acid to bind to at least one probe immobilized on a control detection portion on the solid phase carrier, and
(d) a step of labeling the captured target nucleic acid and control nucleic acid, and detecting signals derived from the target nucleic acid and the control nucleic acid.

The order of carrying out these steps (b), (c) and (d), which are carried out after completion of the step (a), is not particularly limited, and a part of or all of these steps may be simultaneously carried out.

In the present description, the steps (b), (c) and (d) are collectively referred to as a "nucleic acid detection step."

In the nucleic acid detection step, typically, one tag of the two types of tags-added amplification reaction products (target nucleic acids and a control nucleic acid) obtained in the above-described amplification step is first allowed to bind to a labeled probe to form a complex, and thereafter, the other tag is used to capture the complex on a solid phase carrier, so that signals derived from the amplification reaction products are detected.

The probe used in the present invention is a nucleic acid such as a complementary strand reacting with a single-stranded nucleic acid tag. The number of nucleotides in the nucleic acid used as a probe is not particularly limited, and it is, for example, 5 to 80.

The labeled probe used in the present invention is a probe that binds to a labeling substance emitting signals for detection. As such a labeling substance, a conventionally known substance can be selected, as appropriate, and can be used. Examples of the labeling substance include a fluorescent compound, a radioisotope, an electrochemically active compound, colloidal gold particles, colored particles, and coloring agents such as a pigment or a dye. Detection of signals in the step (d) may be carried out by a method that depends on a labeling substance. Detection of signals can be carried out using a measurement device or by visual inspection. For example, when colloidal gold particles are used as labeling substances, a red color generated as a result of agglutination of the colloidal gold particles is detected as signals.

In the present invention, the amplification reaction product (which may be a complex of the amplification reaction product and a labeled probe) is captured on a solid phase carrier by the binding of one tag added to the amplification reaction product with a probe immobilized on the solid phase carrier. The method of immobilizing a probe on the solid phase carrier is not particularly limited in the present invention. For example, a single-stranded nucleic acid probe may be immobilized on the carrier via the 3'-terminus thereof, may also be immobilized thereon via the 5'-terminus thereof, may also be immobilized thereon via a portion other than each-terminal portion thereof, may also be immobilized via one or more portions thereof, or may also be immobilized thereon via a protein. An example of the immobilization method involving the use of a protein is a method of utilizing, what is called, a biotin-avidin reaction, wherein the method comprises coating a solid phase carrier with streptavidin and then immobilizing a biotin-modified probe thereon. The probe immobilized on the solid phase carrier may have a suitable spacer, with respect to the surface of the carrier.

### < Nucleic acid detection device >

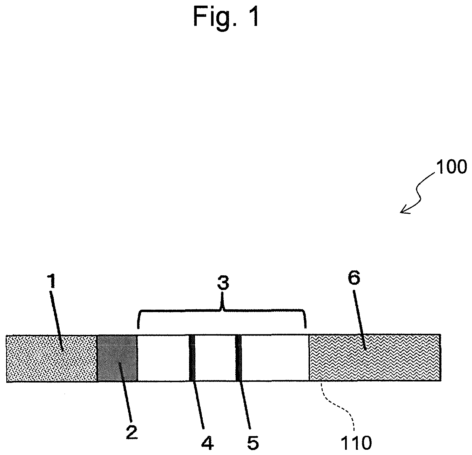

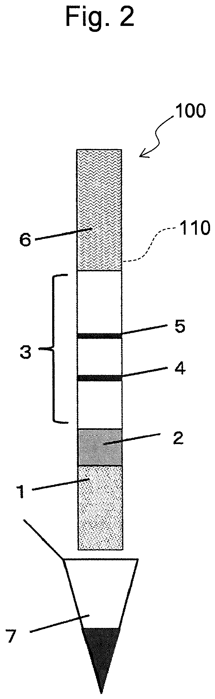

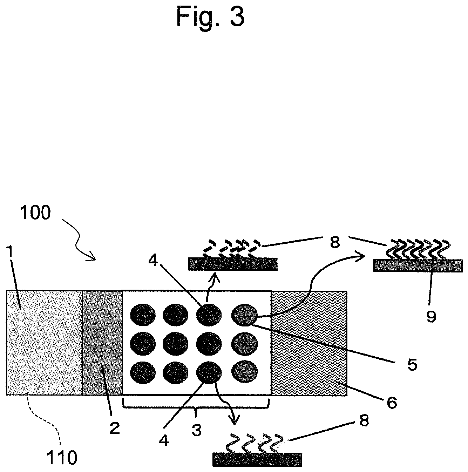

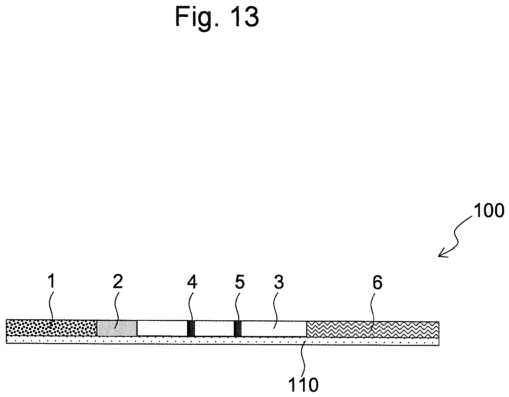

As one embodiment of the nucleic acid detection device of the present invention, a schematic figure of a lateral flow-type device (100) is shown (the plan view is shown in Figure 1 and the cross-sectional view is shown in Figure 13). The shape of the device is not particularly limited, as long as it is able to detect a tagged amplification reaction product. Examples of the shape of the device other than the lateral flow-type device include a dip stick-type device (Figure 2) and an array-type device (Figure 3). The nucleic acid detection devices in these embodiments are all examples of a chromatographic device. Herein, the chromatographic device means a nucleic acid detection device having the shape of a chromatography carrier.

The lateral flow-type device (100) shown in Figure 1 is configured by successively laminating a sample pad (1) to which a tagged amplification reaction product is to be added, a conjugate pad (2) in which a labeled probe is disposed, a solid phase carrier (3) and an absorbent pad (6), on a base material (110) made of plastic. The solid phase carrier (3) has at least one target detection portion (4) and a control detection portion (5), which are each independently disposed. In the present invention, the side of the sample pad is defined as upstream, and the side of the absorbent pad is defined as downstream, for convenience.

In the present invention, the target detection portion (4) mean a portion, in which a probe specifically capturing the tagged amplification reaction product derived from a target nucleic acid is immobilized. On the other hand, the control detection portion (5) means a portion, in which at least one probe specifically capturing target nucleic acid-derived amplification reaction products, as well as a probe specifically capturing the tagged amplification reaction product derived from a control nucleic acid, are immobilized. The type and number of target nucleic acid-derived amplification reaction products captured by the control detection portion are not particularly limited.

In the present invention, the position of the control detection portion is not particularly limited. In the case of a lateral flow-type device or a dip stick-type device, the control detection portion is preferably positioned downstream of the target detection portion.

The sample pad (1), the conjugate pad (2), the solid phase carrier (3) and the absorbent pad (6) each consist of a material such as plastic, glass, cellulose, nitrocellulose, nylon, polyether sulfone, polyvinylidene fluoride, or a porous body such as a filter. The aforementioned components may be constituted with the same material, or may also be constituted with different materials. Moreover, the shape of the carrier is not particularly limited, and it is preferably a tabular shape.

The amplification reaction product to which two types of tags have been added, obtained by the nucleic acid amplification step, is added to the sample pad (1). Regarding the method of adding the amplification reaction product, the reaction solution obtained after completion of the nucleic acid amplification reaction may be directly added dropwise to the sample pad, or the obtained reaction solution may be mixed with a suitable development solution (e.g., a phosphate buffer, a Tris buffer, a Good's buffer, or an SSC buffer) and the mixed solution may be then added dropwise to the sample pad. The development solution can further comprise a surfactant, salts, a protein, a sugar, a nucleic acid, and the like, as necessary. The tagged amplification reaction product added to the sample pad (1) is developed by capillary phenomenon from the direction of the sample pad (1) to the absorbent pad (6), as shown in Figure 1.

The amplification reaction product, to which two types of tags have been added, is allowed to come into contact with a labeled probe, when it is passed through the conjugate pad (2) containing the labeled probe, and the amplification reaction product binds to the labeled probe via one tag thereof.

Subsequently, when the labeled probe-bound amplification reaction product is passed through the solid phase carrier (3), it is captured on the solid phase carrier via the binding of the other tag thereof with a probe immobilized on the solid phase carrier. The control nucleic acid-derived amplification reaction product is captured only in the control detection portion (5) on the solid phase carrier, whereas the target nucleic acid-derived amplification reaction product is captured both in the target detection portion (4) and in the control detection portion (5) on the solid phase carrier. Thereby, in a detection using a nucleic acid detection device of utilizing multiplex PCR, the target nucleic acids are preferentially amplified under conditions in which large quantities of target nucleic acids are present in the reaction system, and even in a case where the amplification reaction of the control nucleic acid is inhibited, target nucleic acid-derived amplification reaction products are captured not only in the target detection portion, but also in the control detection portion, and the products emit signals. Thus, it becomes possible to prevent the disappearance or reduction of signals from the control detection portion.

It is to be noted that the means for achieving the object is not limited only to the means for providing a device in which two or more types of probes are immobilized on a control detection portion. Any means preventing the disappearance or reduction of signals from a control detection portion may be employed. For example, the present technical problem can be solved also by using a device, in which one type of probe is immobilized on the control detection portion. Tagged primers are designed in such a manner that a target nucleic acid is amplified to produce two types of amplified products of the target nucleic acid, namely, a tagged target nucleic acid to be specifically captured on the target detection portion and a tagged target nucleic acid to be specifically captured on the control detection portion, and thus, a multiplex PCR system can be constructed. Thereby, even if there is used a device in which one type of probe is immobilized on the control detection portion, one of the aforementioned two types of amplification reaction products is captured on the detection portion. Therefore, it becomes possible to solve the problem by the aforementioned means.

With regard to the judgment of the results by using the nucleic acid detection device of the present invention, signals derived from the target nucleic acid and the control nucleic acid, which have been subjected to the amplification and/or labeling reactions, are detected using a measurement device or by visual inspection, and the judgment is then carried out based on the obtained results. For example, when colloidal gold particles are used as labeling substances, red coloration occurring with agglutination of the colloidal gold particles is detected as signals, and the judgment is then carried out.

Other shapes of the nucleic acid detection device of the present invention include a dip stick-type nucleic acid detection device (100) shown in Figure 2 and an array-type nucleic acid detection device (100) shown in Figure 3. In addition, it may also be possible that a plurality of array compartments (4,5) are established on the solid phase carrier (3) of the lateral flow-type device (100) or the dip stick-type device (100). In the plurality of array compartments, the same probes may be immobilized, or different probes may also be immobilized. The shape of the nucleic acid detection device can also be designed, while taking into consideration the aspect of capturing and/or detection. For example, in the case of the dip stick-type device (100), the device preferably has a width and a shape, with which the tip of the device (in the illustrated example, the tip on the side of the sample pad (1)) can be immersed in a solution of amplified and/or labeled products, which is to be supplied to a commonly used microtube.

### < Nucleic acid detection kit >

The nucleic acid detection device of the present invention may be a detection kit comprising reagents necessary for nucleic acid detection, such as a control template nucleic acid, a nucleic acid amplification enzyme, a nucleic acid amplification reaction reagent, and a nucleic acid detection reaction reagent, in addition to the nucleic acid detection device. The preservation state of the control template nucleic acid comprised in the present kit is not particularly limited, and it is any state such as a liquid, a frozen product, or a dried product. The detection kit may further comprise the above-described primers.

The nucleic acid amplification reaction reagent comprises components such as a nucleic acid amplification enzyme, a substrate and a buffer. Examples of the substrate include dATP, dTTP, dCTP, dGTP, dUTP, biotin-labeled dCTP, and biotin-labeled dUTP, but are not limited thereto. As a buffer, a buffer containing, for example, magnesium, potassium, a buffer agent, a surfactant, a reducing agent is preferable. The preservation state of the nucleic acid amplification reaction reagent is not particularly limited, and it is any state such as a liquid state, a frozen state, a dried state, or a freeze-dried state.

The nucleic acid detection reaction reagent (nucleic acid detection reagent) is not particularly limited, as long as it is a reagent used in detection. Examples of the nucleic acid detection reaction reagent include the aforementioned development solution, a labeled antibody solution, a coloring substrate solution, and a fluorescent substrate solution, but are not limited thereto. The preservation state may be any state such as a liquid state or a frozen state.

### Examples

Hereinafter, the present invention will be specifically described in the following examples. However, these examples are not intended to limit the technical scope of the present invention.

In the following Examples and Comparative Examples, as a nucleic acid detection device, a nucleic acid detection device 100 having the embodiment shown in Figure 1 was produced and used.

### < Example 1 >

### (1) Production of labeled probe and conjugate pad

5.5 ml of Gold Colloid (40 nm, 9.0 × 10¹⁰ (particles/ml)) (manufactured by British Biocell International) was mixed with 60 µl of 100 µM thiolated DNA (SEQ ID NO: 1), and the obtained mixture was then incubated at 50°C for 16 hours. Sixteen hours later, 250 µl of 0.1 M phosphate buffer (pH 7.0) and 150 µl of 1 M NaCl were added to the reaction mixture, and the thus obtained mixture was then incubated at 50°C for 24 hours. Twenty-four hours later, the reaction mixture was centrifuged (5000 G, 15°C, 20 minutes), and a supernatant was then removed. Thereafter, 6 ml of 5 mM phosphate buffer (pH 7.0) was added to the residue, followed by mixing by inverting. Thereafter, the resultant was centrifuged again (5000 G, 15°C, 20 minutes). After that, 6 ml of a supernatant was removed, and 1.5 ml of 5 mM phosphate buffer (pH 7.0) was then added to the residue. The obtained solution was defined as a labeled probe solution, in which thiolated DNA bound to colloidal gold particles. The prepared solution was added to a glass fiber-made pad, so that the solution became homogeneous, and it was then dried in a vacuum dryer. The resultant was defined as a conjugate pad 2 comprising a labeled probe.

The following thiolated DNA was used in the present step.
- Thiolated DNA: 5'-CTATAAACCCAGTGAAAAATGTTGCCA-SH-3' (SEQ ID NO: 1).

### (2) Production of probe-immobilized solid phase carrier

The nucleic acid detection device 100 used in the present example has a target detection portion 4 and a control detection portion 5, which are disposed in a line from upstream of a solid phase carrier 3.

The target detection portion 4 was produced by applying, in a line, a mixed solution of 200 µl of 100 µM probe A (SEQ ID NO: 2), 200 µl of 2.5 mg/ml streptavidin, 100 µl of 1% BSA solution and 500 µl of 5 mM phosphate buffer to two sites on a nitrocellulose membrane 3 (brand name: Hi-Flow 180, manufactured by Millipore) used as a solid phase carrier, using a dispenser, and then air-drying at 40°C for 30 minutes. The control detection portion 5 was produced by applying, in a line, a mixed solution of 200 µl of 100 µM probe A (SEQ ID NO: 2), 200 µl of 100 µM probe B (SEQ ID NO: 3), 200 µl of 2.5 mg/ml streptavidin, 100 µl of 1% BSA solution and 300 µl of 5 mM phosphate buffer to two sites on the nitrocellulose membrane 3 used as a solid phase carrier, using a dispenser, and then air-drying at 40°C for 30 minutes.

The following probes were used in the present step.
- Probe A: 5'-ATCACACATTAGCTGTCACTCGATGCA-Biotin-3' (SEQ ID NO: 2)
- Probe B: 5'-TCAAAGTCATTGTAAGTCCGTACTAG-Biotin-3' (SEQ ID NO: 3)

### (3) Production of nucleic acid detection device

The nitrocellulose membrane 3 used as a solid phase carrier produced in the above (2) of the present example, the conjugate pad 2 produced in the above (1) of present example, a commonly used sample pad 1 used as a sample addition portion, and an absorbent pad 6 for absorbing the developed sample or labeling substance were adhered to a base material 110 consisting of a backing sheet, so as to produce a nucleic acid detection device 100 capable of detecting an amplification reaction product obtained by the amplification step.

### < Comparative Example 1 >

A nucleic acid detection device 100 was produced by the same method as that applied in Example 1, with the exception that a mixed solution of 200 µl of 100 µM probe B (SEQ ID NO: 3), 200 µl of 2.5 mg/ml streptavidin, 100 µl of 1% BSA solution and 300 µl of 5 mM phosphate buffer was applied in a line to the control detection portion 5. The nucleic acid detection device 100 of Comparative Example 1 has the same structure as that of the nucleic acid detection device 100 of Example 1, with the exception that the probe disposed in the control detection portion 5 was different from that in Example 1.

### < Example 2 >

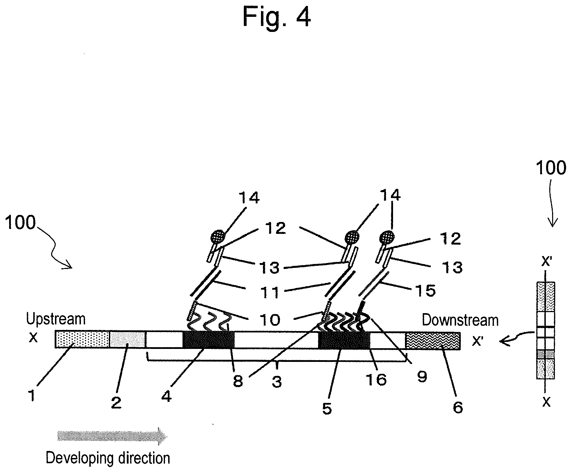

In the present example, pUC19 (manufactured by Takara Bio, Inc.) shown in SEQ ID NO: 4 was employed as a target nucleic acid that is a detection target, and λ phage DNA (manufactured by Eurofins Genomics K. K.) shown in SEQ ID NO: 5 was employed as a control nucleic acid. These nucleic acids were used as templates and subjected to PCR using single-stranded nucleic acid tagged primers. The amplification reaction products were then applied to the device 100 produced in Example 1, and color signals emitted from a complex formed by binding the amplification reaction products with a labeled probe were then detected. The detection is illustrated in Figure 4. In each of Figures 4 to 12 used in the subsequent explanation, a plan view of the nucleic acid device 100 is shown on the right side, and a schematic figure of the X-X' cross-section of the plan view is shown on the left side. In each cross-sectional schematic figure, the base material 110 is omitted, and the ratio of the dimensions of the sample pad 1, the conjugate pad 2, the solid phase carrier 3, the target detection portion 4, the control detection portion 5 and the absorbent pad 6 is appropriately changed for convenience.

### (1) Designing of single-stranded nucleic acid tagged primers

The single-stranded nucleic acid tagged primer used in the present study consists of a primer part able to bind to each nucleic acid to be amplified, and a single-stranded nucleic acid tag that has been added to the 5'-terminal side of the primer part via a polymerase reaction inhibition region (X).

As primer parts for amplifying pUC19, a pUC19 forward primer (SEQ ID NO: 6) and a pUC19 reverse primer (SEQ ID NO: 7) were designed. Likewise, as primer parts for amplifying λ phage DNA, a λ phage DNA forward primer (SEQ ID NO: 12) and a λ phage DNA reverse primer (SEQ ID NO: 13) were designed.

Moreover, to the 5'-terminal side of each primer part, a single-stranded nucleic acid tag was bound via azobenzene used as a polymerase reaction inhibition region (X), so as to design a single-stranded nucleic acid tagged primer. Regarding single-stranded nucleic acid tagged primers for pUC19, a primer T1-X-F1 (SEQ ID NO: 10), in which a single-stranded nucleic acid tag T1 (SEQ ID NO: 8) was added to the 5'-terminal side of the pUC19 forward primer via azobenzene, and a primer T2-X-R1 (SEQ ID NO: 11), in which a single-stranded nucleic acid tag T2 (SEQ ID NO: 9) was added to the pUC19 reverse primer, were designed. Regarding single-stranded nucleic acid tagged primers for λ phage DNA, a primer T3-X-F2 (SEQ ID NO: 15), in which a single-stranded nucleic acid tag T3 (SEQ ID NO: 14) was added to the λ phage DNA forward primer, and a primer T2-X-R2 (SEQ ID NO: 16), in which a single-stranded nucleic acid tag T2 (SEQ ID NO: 9) was added to the λ phage DNA reverse primer, were designed.

The sequences of the primers designed in the present study are as follows.
- pUC19 forward primer: 5'-GGAAACAGCTATGACCATGA-3' (SEQ ID NO: 6)
- pUC19 reverse primer: 5'-tCTATGCGGCATCAGAGCAG-3' (SEQ ID NO: 7)
- Tag sequence T1: 5'-TCGAGTGACAGCTAATGTGTGATT-3' (SEQ ID NO: 8)
- Tag sequence T2: 5'-ATTTTTCACTGGGTTTATAGT-3' (SEQ ID NO: 9)
- Primer T1-X-F1: 5'-TCGAGTGACAGCTAATGTGTGATT X GGAAACAGCTATGACCATGA-3' (SEQ ID NO: 10)
- Primer T2-X-R1: 5'-ATTTTTCACTGGGTTTATAGT X tCTATGCGGCATCAGAGCAG-3' (SEQ ID NO: 11)
- λ phage DNA forward primer: 5'-AAGTTCTCGCTGGAAGAGGT-3' (SEQ ID NO: 12)
- λ phage DNA reverse primer: 5'-AGGATTAGAAGGTCGAACCGT-3' (SEQ ID NO: 13)
- Tag sequence T3: 5'-GTACGGACTTACAATGACTTTGAT-3' (SEQ ID NO: 14)
- Primer T3-X-F2: 5'-GTACGGACTTACAATGACTTTGAT X AAGTTCTCGCTGGAAGAGGT-3' (SEQ ID NO: 15)
- Primer T2-X-R2: 5'-ATTTTTCACTGGGTTTATAGT X AGGATTAGAAGGTCGAACCGT-3' (SEQ ID NO: 16)

It is to be noted that X is represented by the following formula (X).

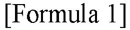

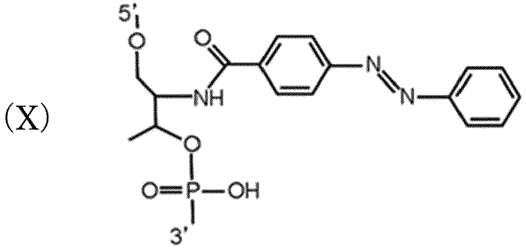

### (2) PCR using single-stranded nucleic acid tagged primers

Using the above-described primer sets, PCR was carried out. 15 pg of the primer set shown in SEQ ID NOs: 10 and 11 and 15 pg of the primer set shown in SEQ ID NOs: 15 and 16, 10 pg of pUC19, and 1 pg of λ phage DNA were added into a 0.2-ml PCR tube, and 100 µl of PCR sample solution (A) was prepared according to the instruction manual of TaKaRa Ex Taq^{(R)} (manufactured by Takara Bio, Inc.). Similarly, PCR sample solution (B) comprising the above-described primer set, 100 pg of pUC19 and 1 pg of λ phage DNA, and PCR sample solution (C) comprising the above-described primer set, 1000 pg of pUC19 and 1 pg of λ phage DNA, were prepared. Thereafter, each tube was equipped into a thermal cycler (GeneAmp PCR System 9700 (manufactured by Applied Biosystem)). The sample solution was subjected to a heat treatment at 95°C for 5 minutes, and was then treated at 95°C for 30 seconds, at 55°C for 30 seconds, and at 72°C for 30 seconds, for 35 cycles, so as to obtain an amplification reaction product. In addition, a solution comprising sterilized water and 1 pg of λ phage DNA, instead of pUC19, was prepared, and PCR was then carried out thereon in the same manner as described above, so as to obtain a negative control (D).

### (3) Detection of amplification reaction product using nucleic acid detection device

The amplification reaction products prepared from the samples (A) to (D) in the above (2) of the present example were not denatured by any denaturing treatment such as heating, but were directly applied to a sample pad 1 of the nucleic acid detection device 100 produced in Example 1, and thereafter, a detection test was carried out. The results were judged by measuring the color intensity of a line using a chromatoreader C10066-10 (manufactured by Hamamatsu Photonics K. K.) 10 minutes after application of each product. The results are shown in Table 1. In addition, the judgment criteria for the measurement values obtained by the chromatoreader are shown in Table 2. In Table 2, the symbol
⊚
   indicates a degree in which the color is extremely dense and thus it can be easily confirmed by visual inspection; the symbol
○
   indicates a degree in which the color is thinner than
⊚
   but it can be easily confirmed by visual inspection; the symbol
△,
   indicates a degree in which the color is thin and confirmation by visual inspection is still possible but slightly difficult; and the symbol
×
   indicates a degree in which the color is not developed and thus confirmation by visual inspection is impossible. In the present descriptions,
⊚
   may be indicated as "4,"
○
   may be indicated as "3,"
Δ
   may be indicated as "2," and
×
   may be indicated as "1."

**[Table 1]**

| | PCR sample | Amount of target nucleic acid template (pg/test) | Amount of control nucleic acid template (pg/test) | Target detection portion signals | Control detection portion signals |
|---|---|---|---|---|---|
| Example 2 | A | 10 | 1 | ⊚ | ⊚ |
| | B | 100 | | ⊚ | ⊚ |
| | C | 1000 | | ⊚ | ⊚ |
| | D | 0 | | × | ⊚ |
| Comparative Example 2 | A | 10 | 1 | ⊚ | ○ |
| | B | 100 | | ⊚ | △ |
| | C | 1000 | | ⊚ | × |
| | D | 0 | | × | ⊚ |

**[Table 2]**

| Determination criteria | Absorbance (mAbs) |
|---|---|
| ⊚ | 100 or more |
| ○ | 40 or more and less than 100 |
| △ | 10 or more and less than 40 |
| × | less than 10 |

### < Comparative Example 2 >

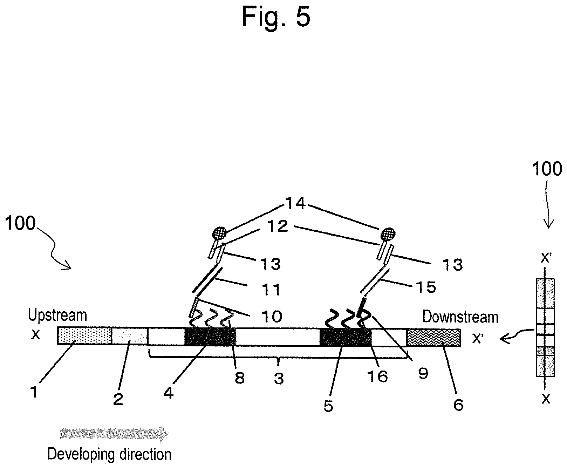

Color signals were detected by the same method as that applied in Example 2, with the exception that the device 100 produced in Comparative Example 1 was used. The detection is illustrated in Figure 5, and the judgment results are shown in Table 1.

Two types of probes 8 and 9, wherein the probe 8 was the same probe as that immobilized on the target detection portion 4, were immobilized on the control detection portion 5 of the device 100 produced in Example 1. Accordingly, signals derived from the target nucleic acid detected in the target detection portion 4 could also be detected in the control detection portion 5. Thereby, it was demonstrated that a reduction in signals from the control detection portion 5 caused by preferential amplification of the target nucleic acids can be prevented, and that signals from the control detection portion 5 can be easily detected even in a case where large quantities of target nucleic acids are present (e.g. 1000 pg). On the other hand, in the case of using the device 100 produced in Comparative Example 1, when large quantities of target nucleic acids were present (e.g. 1000 pg), signals from the control detection portion 5 disappeared, and whether the PCR had been appropriately carried out could not be judged.

### < Example 3 >

### (1) Production of conjugate pad

Using a solution of Gold Colloid (streptavidin conjugate, 80 nm, 9.0 × 10¹⁰ (particles/ml)) (manufactured by Funakoshi Co., Ltd.), which had been 8-fold diluted with 1% BSA solution, a conjugate pad 2 was produced by the same method as that applied in Example 1(1).

### (2) Production of probe-immobilized solid phase carrier

A mixed solution of 200 µl of 100 µM probe A (SEQ ID NO: 2), 200 µl of 1 M NaCl, 100 µl of 1% BSA solution and 500 µl of 5 mM phosphate buffer was applied to a target detection portion 4, and a mixed solution of 200 µl of 100 µM probe A (SEQ ID NO: 2), 200 µl of 100 µM probe B (SEQ ID NO: 3), 200 µl of 1 M NaCl, 100 µl of 1% BSA solution and 300 µl of 5 mM phosphate buffer was applied to a control detection portion 5, and thereafter, each probe-immobilized solid phase carrier 3 was produced by the same method as that applied in Example 1(2).

### (3) Production of nucleic acid detection device

A nucleic acid detection device 100 was produced by the same method as that applied in Example 1(3), using a base material 110 consisting of a backing sheet, a nitrocellulose membrane 3 on which the probe produced in Example 1(2) was immobilized, and the conjugate pad 2 produced in the above (1) of the present example.

### < Comparative Example 3 >

A nucleic acid detection device 100 was produced by the same method as that applied in Example 3, with the exception that a mixed solution of 200 µl of 100 µM probe B (SEQ ID NO: 3), 200 µl of 1 M NaCl, 100 µl of 1% BSA solution and 300 µl of 5 mM phosphate buffer was applied in a line to the control detection portion 5.

### < Example 4 >

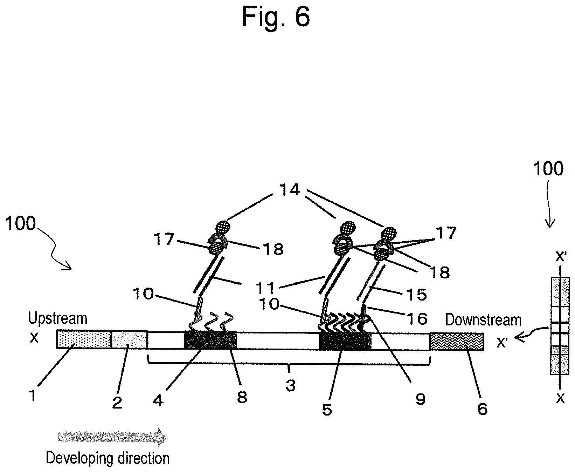

As with Example 2, pUC19 (manufactured by Takara Bio, Inc.) was employed as a target nucleic acid to be detected, and λ phage DNA (manufactured by Eurofins Genomics K. K.) was employed as a control nucleic acid. Using each of these nucleic acids as a template, PCR was carried out with a single-stranded nucleic acid tagged forward primer and a biotin-modified reverse primer. Thereafter, the amplification reaction product was applied to the device 100 produced in Example 3, and color signals emitted from a complex formed by binding the amplification reaction product with a labeled probe were detected. The detection is illustrated in Figure 6.

### (1) Designing of biotin-modified primers and selection of single-stranded nucleic acid tagged primers

As a pUC19 forward primer, the primer shown in SEQ ID NO: 10, which had been designed in Example 2(1), was selected. As a pUC19 reverse primer, a primer Biotinylated-Rl (SEQ ID NO: 17), in which the 5'-terminal side of a primer part was modified with biotin, was designed. As a λ phage DNA forward primer, the primer shown in SEQ ID NO: 15 designed in Example 2(1) was selected. As a λ phage DNA reverse primer, a primer Biotinylated-R2 (SEQ ID NO: 18), in which the 5'-terminal side of a primer part was modified with biotin, was designed.

These single-stranded nucleic acid tagged primers and biotin-modified primers were synthesized by TSUKUBA OLIGO SERVICE CO., LTD., and were then acquired from the company. The primer set designed in the present study is shown below.
- Primer Biotinylated-Rl: 5'-Biotin- tCTATGCGGCATCAGAGCAG-3' (SEQ ID NO: 17)
- Primer Biotinylated-R2: 5'-Biotin- AGGATTAGAAGGTCGAACCGT-3' (SEQ ID NO: 18)

### (2) PCR using biotin-modified primers and single-stranded nucleic acid tagged primers

The samples (A) to (D) were prepared according to the preparation method described in Example 2(2), using the primer set shown in SEQ ID NOs: 10 and 17 and the primer set shown in SEQ ID NOs: 15 and 18, which had been designed in the above (1). Thereafter, PCR was carried out under the same conditions as those in Example 2(2).

### (3) Detection of amplification reaction products using nucleic acid detection device

The amplification reaction products prepared from the samples (A) to (D) in the above (2) of the present example were not denatured by any denaturing treatment such as heating, but were directly applied to a sample pad 1 of the nucleic acid detection device 100 produced in Example 3, and thereafter, a detection test was carried out. The results were judged by measuring the color intensity of a line using a chromatoreader C10066-10 (manufactured by Hamamatsu Photonics K. K.) 10 minutes after application of each product. The results are shown in Table 3.

**[Table 3]**

| | PCR sample | Amount of target nucleic acid template (pg/test) | Amount of control nucleic acid template (pg/test) | Target detection portion signals | Control detection portion signals |
|---|---|---|---|---|---|
| Example 4 | A | 10 | 1 | ⊚ | ⊚ |
| | B | 100 | | ⊚ | ⊚ |
| | C | 1000 | | ⊚ | ⊚ |
| | D | 0 | | × | ⊚ |
| Comparative Example 4 | A | 10 | 1 | ⊚ | ○ |
| | B | 100 | | ⊚ | × |
| | C | 1000 | | ⊚ | × |
| | D | 0 | | × | ⊚ |

### < Comparative Example 4 >

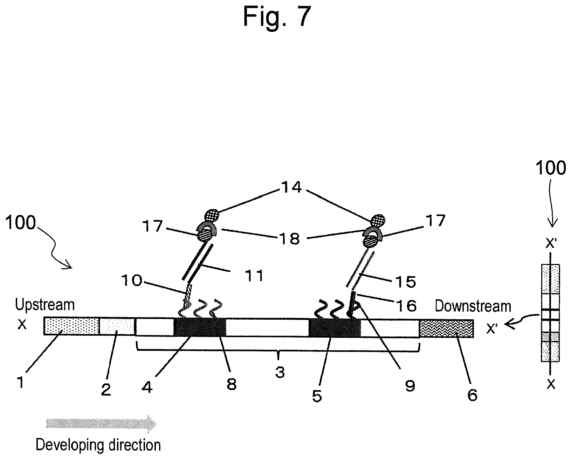

Color signals were detected by the same method as that applied in Example 4, with the exception that the device 100 produced in Comparative Example 3 was used. The detection is illustrated in Figure 7 and the results are shown in Table 3.

Two types of probes 8 and 9, wherein the probe 8 was the same probe as that immobilized on the target detection portion 4, were immobilized on the control detection portion 5 of the device 100 produced in Example 3. Accordingly, signals derived from the target nucleic acid detected in the target detection portion 4 could also be detected in the control detection portion 5. Thereby, it was demonstrated that a reduction in signals from the control detection portion 5 caused by preferential amplification of the target nucleic acids can be prevented, and that signals from the control detection portion 5 can be easily detected even in a case where large quantities of target nucleic acids are present (e.g. 1000 pg). On the other hand, in the case of using the device 100 produced in Comparative Example 3, when large quantities of target nucleic acids were present (e.g. 1000 pg), signals from the control detection portion 5 disappeared, and whether the PCR had been appropriately carried out could not be judged.

### < Example 5 >

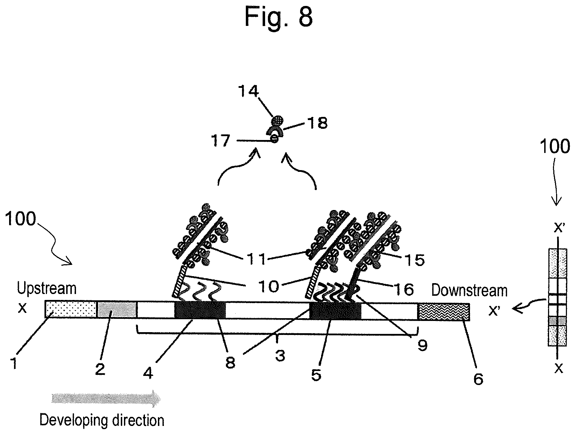

As with Example 2, pUC19 (manufactured by Takara Bio, Inc.) was employed as a target nucleic acid, and λ phage DNA (manufactured by Eurofins Genomics K. K.) was employed as a control nucleic acid. These nucleic acids were used as templates. Using the templates, a primer set, and dNTP comprising biotin-labeled 16-dUTP (manufactured by Roche Applied Science) as a reaction substrate, PCR was carried out. Thereafter, the amplification reaction product was applied to the device 100 produced in Example 3, and color signals emitted from a complex formed by binding the amplification reaction product with a labeled probe were detected. The detection using the device 100 produced in Example 3 is illustrated in Figure 8.

### (1) Selection of single-stranded nucleic acid tagged primers

As a pUC19 forward primer, the primer shown in SEQ ID NO: 10 was selected, and as a pUC19 reverse primer, the primer shown in SEQ ID NO: 7 was selected. On the other hand, as a λ phage DNA forward primer, the primer shown in SEQ ID NO: 15 was selected, and as a λ phage DNA reverse primer, the primer shown in SEQ ID NO: 13 was selected.

### (2) PCR using single-stranded nucleic acid tagged primers and biotin-labeled 16-dUTP

PCR was carried out using the above-described primer set. The samples (A) to (D) were prepared according to the preparation method described in Example 2(2), using the primer set shown in SEQ ID NOs: 7 and 10, the primer set shown in SEQ ID NOs: 13 and 15, dNTP included with TaKaRa Ex Taq^{(R)} (manufactured by Takara Bio, Inc.), and also, biotin-labeled 16-dUTP (manufactured by Roche Applied Science). Thereafter, PCR was carried out under the same conditions as those in Example 2(2).

### (3) Detection of amplification reaction products using nucleic acid detection device

The amplification reaction products prepared from the samples (A) to (D) in the above (2) of the present example were not denatured by any denaturing treatment such as heating, but were directly applied to a sample pad 1 of the nucleic acid detection device 100 produced in Example 3, and thereafter, a detection test was carried out. The results were judged by measuring the color intensity of a line using a chromatoreader C10066-10 (manufactured by Hamamatsu Photonics K. K.) 10 minutes after application of each product. The results are shown in Table 4.

**[Table 4]**

| | PCR sample | Amount of target nucleic acid template (pg/test) | Amount of control nucleic acid template (pg/test) | Target detection portion signals | Control detection portion signals |
|---|---|---|---|---|---|
| Example 5 | A | 10 | 1 | ⊚ | ⊚ |
| | B | 100 | | ⊚ | ⊚ |
| | C | 1000 | | ⊚ | ⊚ |
| | D | 0 | | × | ⊚ |
| Comparative Example 5 | A | 10 | 1 | ⊚ | ○ |
| | B | 100 | | ⊚ | × |
| | C | 1000 | | ⊚ | × |
| | D | 0 | | × | ⊚ |

### < Comparative Example 5 >

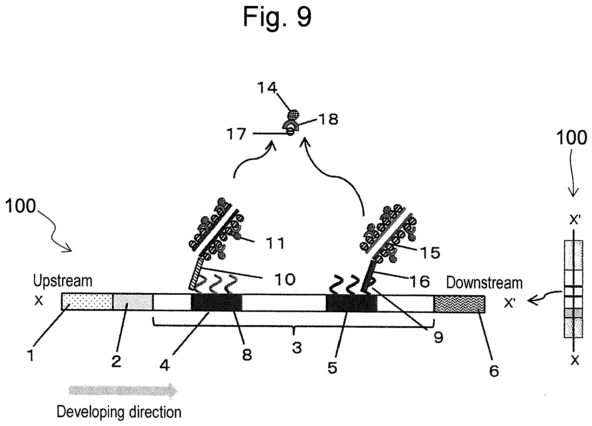

Color signals were detected by the same method as that applied in Example 5, with the exception that the device 100 produced in Comparative Example 3 was used. The detection is illustrated in Figure 9 and the results are shown in Table 4.

Two types of probes 8 and 9, wherein the probe 8 was the same probe as that immobilized on the target detection portion 4, were immobilized on the control detection portion 5 of the device 100 produced in Example 3. Accordingly, signals derived from the target nucleic acid detected in the target detection portion 4 could also be detected in the control detection portion 5. Thereby, it was demonstrated that a reduction in signals from the control detection portion 5 caused by a preferential increase in the target nucleic acids can be prevented, and that signals from the control detection portion 5 can be easily detected even in a case where large quantities of target nucleic acids are present (e.g. 1000 pg). On the other hand, in the case of using the device 100 produced in Comparative Example 3, when large quantities of target nucleic acids were present (e.g. 1000 pg), signals from the control detection portion 5 disappeared, and whether the PCR had been appropriately carried out could not be judged.

### < Example 6 >

### (1) Production of probe-immobilized solid phase carrier

As a probe mixed solution to be used in the target detection portion 4, a solution comprising a 2.0 mg/ml of anti-DIG antibody (manufactured by Roche Applied Science), 2.5% sucrose, and 20 mM TBS (pH 8.0) was prepared. As a probe mixed solution to be used in the control detection portion 5, a solution comprising a 1.0 mg/ml of anti-DIG antibody (manufactured by Roche Applied Science) and an anti-FITC antibody (manufactured by Roche Applied Science), 2.5% sucrose, and 20 mM TBS (pH 8.0) was prepared. Using each of the prepared solutions, a probe-immobilized solid phase carrier 3 was produced by the same method as that applied in Example 1(2).

### (2) Production of nucleic acid detection device

A nucleic acid detection device 100 was produced by the same method as that applied in Example 1(3), using a base material 110 consisting of a backing sheet, the nitrocellulose membrane 3 as a solid phase carrier produced in the above (1) of the present example, and the conjugate pad 2 produced in Example 1(1).

### < Comparative Example 6 >

A nucleic acid detection device 100 was produced by the same method as that applied in Example 6, with the exception that, as a probe mixed solution used in the control detection portion 5, a mixed solution comprising 2.0 mg/ml of anti-FITC antibody (manufactured by Roche Applied Science), 2.5% sucrose and 20 mM TBS (pH 8.0) was applied in a line to the control detection portion 5.

### < Example 7 >

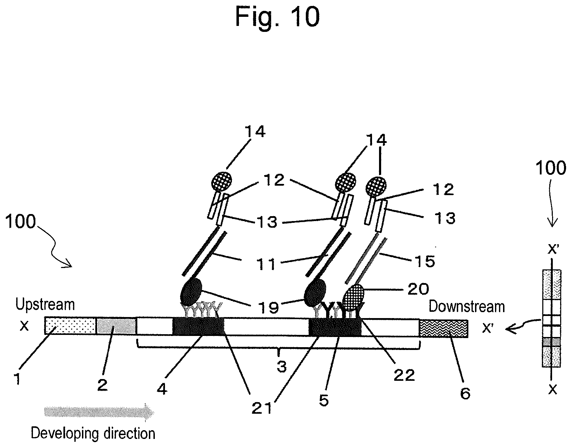

As with Example 2, pUC19 (manufactured by Takara Bio, Inc.) was employed as a target nucleic acid, and λ phage DNA (manufactured by Eurofins Genomics K. K.) was employed as a control nucleic acid. These nucleic acids were used as templates. Using the templates and a primer set, PCR was carried out. Thereafter, the amplification reaction product was applied to the device 100 produced in Example 6, and color signals emitted from a complex formed by binding the amplification reaction product with a labeled probe were detected. The detection using the device 100 produced in Example 6 is illustrated in Figure 10.

### (1) Designing of DIG-modified primers and FITC-modified primers, and selection of single-stranded nucleic acid tagged primers

As a pUC19 forward primer, a primer DIG-F1 (SEQ ID NO: 19), in which the 5'-terminal side of a primer part was modified with DIG, was designed. As a pUC19 reverse primer, the primer shown in SEQ ID NO: 11 was selected. On the other hand, as a λ phage DNA forward primer, a primer FITC-F2 (SEQ ID NO: 20), in which the 5'-terminal side of a primer part was modified with FITC, was designed. As a λ phage DNA reverse primer, the primer shown in SEQ ID NO: 16 was selected.

These DIG-modified primers, FITC-modified primers and single-stranded nucleic acid tagged primers were synthesized by TSUKUBA OLIGO SERVICE CO., LTD., and were then acquired from the company.

The primer set designed in the present study is shown below.
- Primer DIG-F1: 5'-DIG- GGAAACAGCTATGACCATGA-3' (SEQ ID NO: 19)
- Primer FITC-F2: 5'-FITC- AAGTTCTCGCTGGAAGAGGT-3' (SEQ ID NO: 20)

### (2) PCR using DIG-modified primers, FITC-modified primers, and single-stranded nucleic acid tagged primers

PCR was carried out using the above-described primer set. The samples (A) to (D) were prepared according to the preparation method described in Example 2(2), using the primer set shown in SEQ ID NOs: 11 and 19, and the primer set shown in SEQ ID NOs: 16 and 20. Thereafter, PCR was carried out under the same conditions as those in Example 2(2).

### (3) Detection of amplification reaction products using nucleic acid detection device

The amplification reaction products prepared from the samples (A) to (D) in the above (2) of present example were not denatured by any denaturing treatment such as heating, but were directly applied to a sample pad 1 of the nucleic acid detection device 100 produced in Example 6, and thereafter, a detection test was carried out. The results were judged by measuring the color intensity of a line using a chromatoreader C10066-10 (manufactured by Hamamatsu Photonics K. K.) 10 minutes after application of each product. The results are shown in Table 5.

**[Table 5]**

| | PCR sample | Amount of target nucleic acid template (pg/test) | Amount of control nucleic acid template (pg/test) | Target detection portion signals | Control detection portion signals |
|---|---|---|---|---|---|
| Example 7 | A | 10 | 1 | ⊚ | ⊚ |
| | B | 100 | | ⊚ | ⊚ |
| | C | 1000 | | ⊚ | ⊚ |
| | D | ⊚ | | × | ⊚ |
| Comparative Example 7 | A | 10 | 1 | ⊚ | ○ |
| | B | 100 | | ⊚ | △ |
| | C | 1000 | | ⊚ | × |
| | D | 0 | | × | ⊚ |

### < Comparative Example 7 >

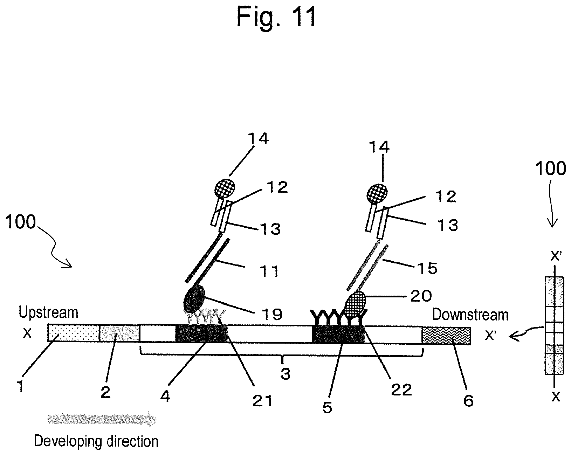

Color signals were detected by the same method as that applied in Example 6, with the exception that the device 100 produced in Comparative Example 6 was used. The detection is illustrated in Figure 11, and the judgment results are shown in Table 5.

Two types of probes 21 and 22, wherein the probe 22 was the same probe as that immobilized on the target detection portion 4, were immobilized on the control detection portion 5 of the device 100 produced in Example 6. Accordingly, signals derived from the target nucleic acid detected in the target detection portion 4 could also be detected in the control detection portion 5. Thereby, it was demonstrated that a reduction in signals from the control detection portion 5 caused by a preferential increase in the target nucleic acids can be prevented, and that signals from the control detection portion 5 can be easily detected even in a case where large quantities of target nucleic acids are present (e.g. 1000 pg). On the other hand, in the case of using the device 100 produced in Comparative Example 6, when large quantities of target nucleic acids were present (e.g. 1000 pg), signals from the control detection portion 5 disappeared, and whether the PCR had been appropriately carried out could not be judged.

### < Example 8 >

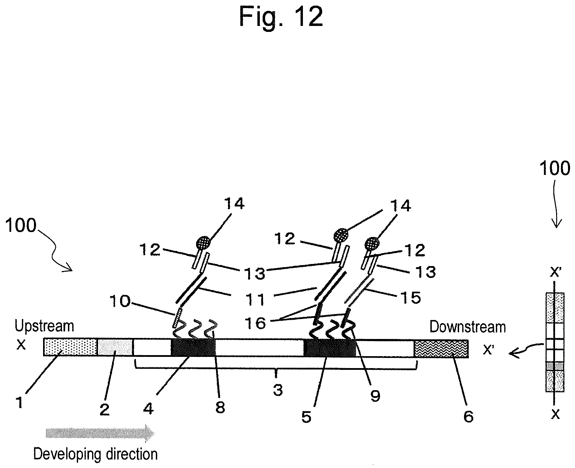

In the present example, we did not apply the method of immobilizing two types of probes, namely, a probe for capturing a control nucleic acid and a probe for capturing a target nucleic acid, on a control detection portion 5, which was applied in the previous examples. In the present example, we constructed an amplification reaction system of producing two types of amplified products of a target nucleic acid having different types of tags, so that we prevented a reduction in signals from the control detection portion 5. Specifically, tagged primers were designed, such that two types of amplified products of a target nucleic acid, namely, a target nucleic acid having a tag to be specifically captured on a target detection portion 4 and a target nucleic acid having a tag to be specifically captured on a control detection portion 5 were amplified, and such that one of the two types of amplified products is captured on the control detection portion 5. The detection of the present example is illustrated in Figure 12. As shown in Figure 12, the amplification reaction product of either one of the aforementioned two types of target nucleic acids was captured on the control detection portion 5.

As with Example 2, pUC19 (manufactured by Takara Bio, Inc.) was used as a target nucleic acid, and λ phage DNA (manufactured by Eurofins Genomics K. K.) was used as a control nucleic acid. After PCR had been carried out, the amplification reaction product was applied to the device 100 produced in Comparative Example 1, and color signals emitted from a complex formed by binding the amplification reaction product with a labeled probe were detected.

### (1) Designing and selection of single-stranded nucleic acid tagged primers

In the present study, two types of primer sets were prepared as pUC19 primer sets. As a first primer set, primers shown in SEQ ID NOs: 10 and 11 were selected. In addition, a primer T3-X-F1 (SEQ ID NO: 21) was designed by adding a single-stranded nucleic acid tag T3 (SEQ ID NO: 14) to the 5'-terminal side of the forward primer shown in SEQ ID NO: 6 via azobenzene used as a polymerase reaction inhibition region (X). Thus, as a second primer set, primers shown in SEQ ID NOs: 11 and 21 were selected. As a λ phage DNA primer set, a primer set shown in SEQ ID NOs: 15 and 16 was selected. These single-stranded nucleic acid tagged primers were synthesized by TSUKUBA OLIGO SERVICE CO., LTD., and were then acquired from the company.

The primer designed in the present study is shown below.
- Primer T3-X-F1 : 5'-GTACGGACTTACAATGACTTTGAT X GGAAACAGCTATGACCATGA-3' (SEQ ID NO: 21)

### (2) PCR using single-stranded nucleic acid tagged primers

The samples (A) to (D) were prepared according to the preparation method described in Example 2(2), using the three types of primer sets, namely, the primer set shown in SEQ ID NOs: 10 and 11, the primer set shown in SEQ ID NOs: 11 and 21, the primer set shown in SEQ ID NOs: 15 and 16, which had been designed in the above (1) of the present example. Using these samples, PCR was carried out under the same conditions as those applied in Example 2(2).

### (3) Detection of amplification reaction products using nucleic acid detection device

The amplification reaction products prepared from the samples (A) to (D) in the above (2) of the present example were not denatured by any denaturing treatment such as heating, but were directly applied to a sample pad 1 of the nucleic acid detection device 100 produced in Comparative Example 1, and thereafter, a detection test was carried out. The results were judged by measuring the color intensity of a line using a chromatoreader C10066-10 (manufactured by Hamamatsu Photonics K. K.) 10 minutes after application of each product. The results are shown in Table 6.

**[Table 6]**

| | PCR sample | Amount of target nucleic acid template (pg/test) | Amount of control nucleic acid template (pg/test) | Target detection portion signals | Control detection portion signals |
|---|---|---|---|---|---|
| Example 8 | A | 10 | 1 | ⊚ | ⊚ |
| | B | 100 | | ⊚ | ⊚ |
| | C | 1000 | | ⊚ | ⊚ |
| | D | 0 | | × | ⊚ |
| Comparative Example 8 | A | 10 | 1 | ⊚ | ○ |
| | B | 100 | | ⊚ | △ |
| | C | 1000 | | ⊚ | × |
| | D | 0 | | × | ⊚ |

### < Comparative Example 8 >

Color signals were detected by the same method as that applied in Comparative Example 2. The detection is illustrated in Figure 5 and the results are shown in Table 6.

In Example 8, two types of target nucleic acids, namely, a target nucleic acid having a tag to be specifically captured on a target detection portion 4 and a target nucleic acid having a tag to be specifically captured on a control detection portion 5, were prepared. As a detection device 100, a device, in which one type of probe was immobilized both on the target detection portion 4 and on the control detection portion 5, was used. Also in the present example, target nucleic acids were detected not only in the target detection portion 4, but also in the control detection portion 5. Thus, as shown in Table 6, signals derived from the target nucleic acids could be detected in the control detection portion 5, even under conditions in which 1000 pg of the target nucleic acids and 1 pg of the control nucleic acid were present. Therefore, it was demonstrated that the disappearance or reduction of signals in the control detection portion 5 can be prevented by preparing target nucleic acids having two types of tags. On the other hand, in Comparative Example 8, when large quantities of target nucleic acids were present (1000 pg), signals from the control detection portion 5 disappeared, and whether PCR had been appropriately carried out could not be judged.

As given above, the invention of the present application is described with reference to embodiments and comparative embodiments. However, the present invention is not limited to the above-described embodiments and comparative embodiments. The present embodiment can also be carried out by applying a detection method using the dip stick-type device 100 or the array-type device 100, or a detection method comprising subjecting the amplified and/or labeled product to a heat treatment and then detecting it in the form of a single strand, or a method of using a sugar chain as a tag and using lection as a probe to be immobilized on a carrier.

### Reference Signs List

1. Sample pad
2. Conjugate pad
3. Probe-retaining carrier
4. Target detection portion
5. Control detection portion
6. Absorbent pad
7. Sample-containing tube
8. Target nucleic acid-capturing probe
9. Control nucleic acid-capturing probe
10. Tag sequence shown in SEQ ID NO: 8
11. Target nucleic acid amplification reaction product
12. Thiolated DNA shown in SEQ ID NO: 1
13. Tag sequence shown in SEQ ID NO: 9
14. Colloidal gold particles
15. Control nucleic acid amplification reaction product
16. Tag sequence shown in SEQ ID NO: 14
17. Biotin
18. Streptavidin
19. 5'-terminus binding DIG
20. 5'-terminus binding FITC
21. Target nucleic acid-capturing anti-DIG antibody
22. Control nucleic acid-capturing anti-FITC antibody
100. Device
110. Backing sheet

## Claims

1. A nucleic acid detection device comprising at least one target detection portion and a control detection portion, wherein a probe for capturing a target nucleic acid is immobilized on the target detection portion, and a probe for capturing a control nucleic acid and at least one probe for capturing the target nucleic acid are immobilized on the control detection portion,
wherein the target nucleic acid and the control nucleic acid are amplification reaction products that have undergone an amplification step,
wherein the probe is a nucleic acid.

2. The nucleic acid detection device according to claim 1, which is a chromatographic device.

3. A nucleic acid detection kit comprising the nucleic acid detection device according to claim 1 or 2, a control template nucleic acid, a nucleic acid amplification enzyme, a nucleic acid amplification reaction reagent, and a nucleic acid detection reaction reagent.

4. A nucleic acid detection method for detecting at least one target nucleic acid and a control nucleic acid, which comprises the following steps (a) to (d):
(a) a step of simultaneously performing a nucleic acid amplification reaction in a single reaction vessel, using primers, a template nucleic acid and a nucleic acid amplification reaction reagent, so as to amplify the at least one target nucleic acid and the control nucleic acid,
(b) a step of allowing the at least one target nucleic acid to bind to a probe immobilized on a target detection portion on a solid phase carrier,
(c) a step of allowing the at least one target nucleic acid and the control nucleic acid to bind to at least one probe immobilized on a control detection portion on the solid phase carrier, and
(d) a step of labeling the captured target nucleic acid and control nucleic acid, and detecting signals derived from the target nucleic acid and the control nucleic acid, wherein the probe is a nucleic acid.

## Patentansprüche

1. Vorrichtung zum Nachweis einer Nucleinsäure, die mindestens einen Target-Nachweisteil und einen Kontroll-Nachweisteil umfasst, wobei eine Sonde zum Einfangen der Target-Nucleinsäure auf dem Target-Nachweisteil immobilisiert ist, und eine Sonde zum Einfangen einer Kontroll-Nucleinsäure und mindestens eine Sonde zum Einfangen der Target-Nucleinsäure auf dem Kontroll-Nachweisteil immobilisiert sind,
wobei die Target-Nucleinsäure und die Kontroll-Nucleinsäure Amplifikationsreaktionsprodukte sind, die einem Amplifikationsschritt unterzogen wurden,
wobei die Sonde eine Nucleinsäure ist.

2. Vorrichtung zum Nachweis einer Nucleinsäure nach Anspruch 1, die eine chromatographische Vorrichtung ist.

3. Nucleinsäure-Nachweis-Kit, das die Vorrichtung zum Nachweis einer Nucleinsäure nach Anspruch 1 oder 2 umfasst, eine Kontroll-Template-Nucleinsäure, ein Nucleinsäure-Amplifikation-Enzym, ein Nucleinsäure-Amplifikationsreaktion-Reagens und ein Nucleinsäure-Nachweisreaktion-Reagens.

4. Verfahren zum Nachweis einer Nucleinsäure zum Nachweisen mindestens einer Target-Nucleinsäure und einer Kontroll-Nucleinsäure, das die folgenden Schritte (a) bis (d) umfasst:
(a) einen Schritt des gleichzeitigen Durchführens einer Nucleinsäure-Amplifikationsreaktion in einem einzelnen Reaktionsbehälter unter Verwendung von Primern, einer Template-Nucleinsäure und eines Nucleinsäure-Amplifikationsreaktion-Reagens, um die mindestens eine Target-Nucleinsäure und die Kontroll-Nucleinsäure zu amplifizieren,
(b) einen Schritt des Ermöglichens, dass die mindestens eine Target-Nucleinsäure an eine Sonde bindet, die auf dem Target-Nachweisteil auf einem Träger solider Phase immobilisiert ist,
(c) einen Schritt des Ermöglichens, dass die mindestens eine Target-Nucleinsäure und die Kontroll-Nucleinsäure an mindestens eine Sonde binden, die auf dem Kontroll-Nachweisteil auf dem Träger solider Phase immobilisiert ist, und
(d) einen Schritt des Markierens der eingefangenen Target-Nucleinsäure und Kontroll-Nucleinsäure und des Nachweisens der Signale, die von der Target-Nucleinsäure und der Kontroll-Nucleinsäure stammen,
wobei die Sonde eine Nucleinsäure ist.

## Revendications

1. Dispositif de détection d'acide nucléique comprenant au moins une partie de détection de cible et une partie de détection de témoin, dans lequel une sonde pour capturer un acide nucléique cible est immobilisée sur la partie de détection de cible, et une sonde pour capturer un acide nucléique témoin et au moins une sonde pour capturer l'acide nucléique cible sont immobilisées sur la partie de détection de témoin,
dans lequel l'acide nucléique cible et l'acide nucléique témoin sont des produits de réaction d'amplification qui ont subi une étape d'amplification,
dans lequel la sonde est un acide nucléique.

2. Dispositif de détection d'acide nucléique selon la revendication 1, qui est un dispositif chromatographique.

3. Kit de détection d'acide nucléique comprenant le dispositif de détection d'acide nucléique selon la revendication 1 ou 2, un acide nucléique témoin matrice, une enzyme d'amplification d'acide nucléique, un réactif de réaction d'amplification d'acide nucléique, et un réactif de réaction de détection d'acide nucléique.

4. Méthode de détection d'acide nucléique pour détecter au moins un acide nucléique cible et un acide nucléique témoin, qui comprend les étapes (a) à (d) suivantes :
(a) une étape de mise en oeuvre simultanée d'une réaction d'amplification d'acide nucléique dans un seul récipient réactionnel, utilisant des amorces, un acide nucléique matrice et un réactif de réaction d'amplification d'acide nucléique, de façon à amplifier l'au moins un acide nucléique cible et l'acide nucléique témoin,
(b) une étape consistant à laisser l'au moins un acide nucléique cible se lier à une sonde immobilisée sur une partie de détection de cible sur un support en phase solide,
(c) une étape consistant à laisser l'au moins un acide nucléique cible et l'acide nucléique témoin se lier à au moins une sonde immobilisée sur une partie de détection de témoin sur le support en phase solide, et
(d) une étape de marquage de l'acide nucléique cible et de l'acide nucléique témoin capturés, et de détection de signaux dérivés de l'acide nucléique cible et de l'acide nucléique témoin,
dans laquelle la sonde est un acide nucléique.
